# EUROPEAN PATENT APPLICATION

(11) **EP 2 050 383 A1**
(43) Date of publication of application: **22.04.2009**
(21) Application number: 07792294.6
(22) Date of filing: 09.08.2007
(51) Int. Cl.: A61B 1/00, G02B 23/24

(54) **OPERATION DEVICE AND OPERATION DEVICE FOR ENDOSCOPE**

(30) Priority: 10.08.2006 JP 2006218507
(71) Applicant: Olympus Medical Systems Corp., Tokyo 151-0072 (JP)
(72) Inventor: KOITABASHI, Masanobu c/o Intellectual Property Support Department OLYMPUS INTELLECTUAL PROPERTY SERVICES CO., LTD., Hachioji-shi Tokyo 192-8512 (JP)
(74) Representative: von Hellfeld, Axel
(86) International application number: PCT/JP2007/065644
(87) International publication number: WO 2008/018554

(57) **Abstract**

A central neutral position (N position) in a rotational movement range of a stick shaft (61) of a joystick apparatus (45) attached to a main body part (52) of a remote controller operation portion (12) and rotationally moving about a rotational movement supporting point (O) is set to a tilt angle (θ) tilted toward a grip portion (51) by a predetermined angle and the joystick apparatus (45) is arranged to be tilted to the side of the grip portion (51), so that it is made easy for a finger of an operator to reach the stick shaft (61) of the joystick apparatus (45) even in a state that the operator has grasped the grip portion (51).

## Description

### Technical Field

The present invention relates to an operation apparatus constituted to operate an endoscope provided with a motorized bending portion and an operation apparatus for an endoscope.

### Background Art

Generally, in an endoscope provided with a motorized bending portion, a controller for an endoscope having a configuration using a joystick has been developed as bending operation input means for controlling the motorized bending portion. In a controller for an endoscope described in US Patent Application Publication No. 2005/0075538 (Pat. Document 1), a grasping portion grasped by an operation and an operation portion are arranged. In the operation portion, various operation buttons and a joystick are arranged. In the controller for a motorized bending endoscope using the joystick as bending operating means in this manner, the motorized bending portion can be operated in a bending manner in a desired direction of an upward direction, a downward direction, a leftward direction, and a rightward direction according to only operation of the joystick in the upward, downward, leftward, or rightward direction.

A stick shaft of the joystick is tilted to an arbitrary direction from a neutral position (an inclination angle is 0°) around a rotational movement point. The maximum inclination angles at inclination times are set to about 30°, respectively, for example. The maximum bending angle of the bending portion bending according to tilting action of the stick shaft of the joystick is set to about 180° or 160°, for example. At this time, the tilting action angle of the stick shaft of the joystick and the bending angle of the bending portion are set to be proportional to each other. Therefore, the bending angle of the bending portion is set to become larger than the tilting action angle of the stick shaft of the joystick. For example, when the tilting angle of the stick shaft of the joystick is 1°, the bending portion is operated in a bending manner with a bending angle of about 6°.

### Disclosure of Invention

When the joystick is used in the controller for an endoscope as the bending operation input means, the bending angle of the bending portion becomes larger than the tilting action angle of the stick shaft of the joystick. For example, when 30° of a tilting angle of the stick shaft of the joystick corresponds to 180° of the maximum bending angle of the bending portion, the bending angle of the bending portion reaches as much as 6° for each 1° of the tilting angle of the stick shaft of the joystick. Therefore, a delicate operation of the stick shaft of the joystick is required.

By elongating a length of the stick shaft of the joystick (a length between a rotation center of the stick shaft of the joystick and a distal end portion thereof), a working stroke of the distal end portion of the stick shaft of the joystick can be elongated. In this case, operation of the joystick can be made easy. However, when the working stroke of the stick shaft of the joystick is made large, such a tendency occurs that, when an operator tilts the controller relative to a grip portion (a grasping portion) grasped by the operator in a far direction to the maximum, the stick shaft of the joystick becomes far beyond the grip portion. In this case, there is a possibility that a finger of the operator does not reach the stick shaft in a state that he/she has grasped the grip portion. Therefore, when the operator tries to move the stick shaft of the joystick in a direction in which the grip portion moves far from him/her, it is difficult for the operator to conduct such an operation.

Further, there are many cases where an upper surface of the stick shaft of the joystick is formed in a flat surface shape. In this case, since the stick shaft of the joystick is tilted around a rotation center thereof, the upper surface of the stick shaft of the joystick faces in the opposite direction to a hand of the operator in a state that the stick shaft of the joystick has been tilted to the grip portion in a far direction to the maximum. Therefore, there is a possibility that a finger of the operator does not reach the upper surface of the stick shaft of the joystick.

In view of these circumstances, the present invention has been made, and an object thereof is to provide an operation apparatus configured to be operated easily even when an operation member such as a joystick is moved in a direction in which it is moved far from the grasping portion, and an operation apparatus for an endoscope.

According to a first aspect of the present invention, an operation apparatus comprising: a main body part including an operation member whose proximal end is pivoted via a rotational movement supporting point so as to be capable of rotationally moving and which is supported so as to be tiltable between a central neutral position in a rotational movement range where the operation member rotationally moves about the rotational movement supporting point and an tilt position tilted from the neutral position; a gasping portion which can be grasped by an operator; an action member which acts in response to tilting of the operation member; and an output portion which outputs an instruction signal changing according to action of the action member to an external apparatus, wherein the operation member is configured such that the neutral position is set at a tilt angle tilted toward the grasping portion by a predetermined angle.

With the configuration, the proximal end of the operation member of the main body part is rotatably pivoted via the rotational movement supporting point, and the operation member of the main body part is supported so as to be capable of tilting between a neutral portion positioned at the center of a rotational movement range where the operation member rotationally moves about the rotational movement supporting point and a tilt position tilted from the neutral position. Since the neutral position is set at the tilt angle tilted toward the grasping portion grasped by an operator by the predetermined angle, it is made easy for a finger of a hand of the operator being grasping the grasping portion to reach the operation member even in the maximum tilted position of the operation member where the operation member has been tilted in a direction far from the grasping portion to the maximum, which results in easiness of the operation.

Preferably, the tilt angle is set such that an angle defined by the center line of the operation member and the center line of the grasping portion is an angle smaller than 90° at the neutral position.

With the configuration, by setting the tilt angle at which the neutral position of the operation member is set such that the angle defined by the center line of the operation member and the center line of the grasping portion is an angle smaller than 90° at the neutral position, it is made easy for a finger of a hand of the operator being grasping the grasping portion to reach the operation member even in the maximum tilt position of the operation member where the operation member has been tilted in a direction far from the grasping portion to the maximum, which results in easiness of the operation.

Preferably, the grasping portion includes a flat-shaped first receiving portion receiving a root portion of the thumb of the operator and a second receiving portion disposed on a side surface opposite to the first receiving portion and receiving a finger of a hand of the operator other than the thumb, and the first receiving portion is disposed on the side of a mount surface for the operation member of the main body part.

With the configuration, by causing the flat-shaped first receiving portion of the grasping portion to receive a root portion of a thumb of a hand of an operator, causing the second receiving portion disposed on a side surface opposite to the first receiving portion to receive a finger(s) of the hand other than the thumb, and disposing the first receiving portion on the side of the mount surface of the operation member of the main body part, it is made easy for the thumb of the hand of the operator grasping the grasping portion to reach the operation member, which results in easiness of the operation.

Preferably, the main body part is configured such that a mount surface of operating means other than the operation member is disposed on a side surface opposite to the mount surface for the operation member.

With the configuration, by disposing operating means other than the operation member on a mount surface positioned on the side of the opposite side to the mount surface for the operation member of the main body part, the operation member is operated by the thumb of the hand of the operator while the operating means other than the operation member can be operated by a finger or fingers other than the thumb.

Preferably, a main body part having operating means for operating a movable member; a grasping portion which can be grasped by an operator; an action member which acts in response to operation of the operating means; and an output portion which outputs an instruction signal changing according to action of the action member to an external apparatus, wherein the main body part is configured such that a mount surface for the operating means is set at a tilt angle tilted toward the grasping portion by a predetermined angle.

With the configuration, by setting the mount surface for the operating means of the main body part at the tilt angle tilted toward the grasping portion by the predetermined angle, it is made easy for the finger of the hand of the operator grasping the grasping portion to reach the operating means, which results in easiness of the operation of the operating means.

Preferably, the tilt angle is set such that an angle defined by a flat surface of the mount surface for the operating means and the center line of the grasping portion is an angle smaller than 90°.

With the configuration, by setting the tilt angle of the mount surface for the operating means of the main body part such that an angle defined by a plane of the mount surface for the operating means and the center line of the grasping portion is an angle smaller than 90°, it is made easy for the finger of the hand of the operator grasping the grasping portion to operate the operating means.

Preferably, the grasping portion includes a flat-shaped first receiving portion receiving a root portion of the thumb of the operator and a second receiving portion disposed on a side surface opposite to the first receiving portion and receiving a finger of the operator other than the thumb, and the main body part has a first mount surface arranged on a side surface positioned on the side of the first receiving portion and a second mount surface arranged on a side surface positioned on the side of the second receiving portion.

With the configuration, since a root portion of a thumb of a hand of an operator is received by the flat-shaped first receiving portion of the grasping portion, a finger(s) of the hand other than the thumb is received by the second receiving portion arranged on a side surface opposite to the first receiving portion, the first mount surface of the main body part is disposed on a side surface positioned on the side of the first receiving portion, and the second mount surface is disposed on a side surface on the side of the second receiving portion, it is made easy for the finger of the hand of the operator to reach the operating means of the main body part, which results in easiness of the operation.

Preferably, one of a trackball and an operation button indicating a bending direction is disposed on the first mount surface.

With the configuration, by arranging either one of the trackball or the operation button indicating the bending direction on the first mount surface of the main body part, it is made easy for the finger of the hand of the operator grasping the grasping portion to reach the trackball or the operation button of the main body part, which results in easiness of the operation.

According to second aspect of the present invention, an operation apparatus for an endoscope having a bending operation apparatus for issuing an action instruction to a drive apparatus connected to a bending portion of the endoscope, comprising: a main body part including an operation member whose proximal end is pivoted via a rotational movement supporting point in a rotational moving manner and which is supported so as to be tiltable between a central neutral position in a rotational movement range where the operation member rotationally moves about the rotational movement supporting point and a tilt position tilted from the neutral position; a gasping portion which can be grasped by an operator; an action member which acts in response to tilting of the operation member; and an output portion which outputs an instruction signal changing according to action of the action member to a bending control apparatus controlling the drive apparatus, wherein the operation member is configured such that the neutral position is set at a tilt angle tilted toward the grasping portion by a predetermined angle.

With the configuration, the proximal end of the operation member of the main body part is pivoted so as to be rotationally movable via the rotational movement supporting point, and the operation member of the main body part is supported so as to be capable of tilting between a neutral portion positioned at the center of a rotation range where the operation member rotationally moves about the rotational movement supporting point and a tilt position tilted from the neutral position. Since the neutral position of the operation member is set at the tilt angle tilted toward the grasping portion grasped by an operator by the predetermined angle, it is made easy for a finger of a hand of the operator being grasping the grasping portion to reach the operation member even in the maximum tilt position of the operation member where the operation member has been tilted in a direction far from the grasping portion to the maximum at an operation time of the bending portion of the endoscope, which results in easiness of the operation.

Preferably, the tilt angle is set such that an angle defined by the center line of the operation member and the center line of the grasping portion is an angle smaller than 90° at the neutral position.

With the configuration, by setting the tilt angle at which the neutral position of the operation member is set such that the angle defined by the center line of the operation member and the center line of the grasping portion is an angle smaller than 90° at the neutral position, it is made easy for a finger of a hand of the operator being grasping the grasping portion to reach the operation member even in the maximum tilt position of the operation member where the operation member has been tilted in a direction far from the grasping portion to the maximum at an operation time of the bending portion of the endoscope, which results in easiness of the operation.

Preferably, the grasping portion includes a flat-shaped first receiving portion receiving a root portion of the thumb of the operator and a second receiving portion disposed on a side surface opposite to the first receiving portion and receiving a finger of the operator other than the thumb, and the first receiving portion is disposed on the side of a mount surface for the operation member of the main body part.

With the configuration, by causing the flat-shaped first receiving portion of the grasping portion to receive a root portion of a thumb of a hand of an operator, causing the second receiving portion disposed on a side surface opposite to the first receiving portion to receive a finger(s) of the hand other than the thumb, and disposing the first receiving portion on the side of the mount surface for the operation member of the main body part, it is made easy for the thumb of the hand of the operator grasping the grasping portion to reach the operation member at an operation time of the bending portion of the endoscope, which results in easiness of the operation.

Preferably, the main body part is configured such that a second mount surface on which operating means other than the operation member is mounted is disposed on a side surface positioned on the side opposite to a mount surface on which the operation member is mounted.

With the configuration, by arranging operating means other than the operation member on a mount surface positioned on the opposite side to the mount surface for the operation member of the main body part, the operation member is operated by the thumb of the hand of the operator at an operation time of the bending portion of the endoscope while the operating means other than the operation member can be operated by a finger other than the thumb.

Preferably, the second mount surface includes a suction switch controlling suction action of the endoscope, a gas-feed and water-feed switch controlling gas-feed and water-feed actions of the endoscope, and a video switch of the endoscope.

With the configuration, an suction action of the endoscope is controlled by the suction switch on the second mount surface, a gas-feed/water-feed action of the endoscope is controlled by the gas-feed and water-feed switch, and photographing of the endoscope is controlled by the video switch.

Preferably, the operation member comprises a joystick instructing a bending direction of the bending portion, the joystick has a finger receiving portion at a head portion of a stick shaft, and the finger receiving portion is formed with a projecting portion arc-shaped along a direction corresponding to upward and downward bending directions of the bending portion and a recessed portion arc-shaped along directions corresponding to leftward and rightward bending directions of the bending portion.

With the configuration, since the bending direction of the bending portion is indicated by the joystick of the operation member, the arc-shaped projecting portion of the finger receiving portion at the head portion of the stick shaft of the joystick is formed along a direction corresponding to an up-and-down bending direction of the bending portion, confirmation of the upward and downward bending directions of the bending portion is made easy, a finger of an operator reaches the upper surface of the finger receiving portion even when the joystick has been tilted in a direction far from the grasping portion to the maximum at an operation time of the bending portion of the endoscope, and the finger of the operator reaches the upper surface of the finger receiving portion of the joystick even when the joystick has been tilted in a direction close to the grasping portion. Further, by forming an arc-shaped recessed portion along a direction corresponding to a left-and-right direction of the bending portion, confirmation of the leftward and rightward bending directions of the bending portion is made easy and the finger of the operator contacts with an upper ridge line of the recessed portion of the finger receiving portion at an operation time of the bending portion of the endoscope, which results in easiness of leftward and rightward operations of the joystick.

Preferably, the finger receiving portion has a rib for finger rest formed by further projecting a portion of the arc-shaped projecting portion in the arc-shaped recessed portion.

With the configuration, by providing a rib for finger rest formed by further protruding a portion of the arc-shaped projecting portion in the arc-shaped recessed portion of the finger receiving portion, it is made easy for the finger of the operator to catch the finger receiving portion, which results in easiness of operation of the bending direction in the upward and downward directions of the bending portion.

According to third aspect of the present invention, an operation apparatus for an endoscope having a bending operation apparatus for issuing an action instruction to a drive apparatus connected to a bending portion of the endoscope, comprising: a main body part including operating means for instructing a bending direction of the bending portion; a grasping portion which can be grasped by an operator; an action member which acts in response to operation of the operating means; and an output portion which outputs an instruction signal changing according to action of the action member to a bending control apparatus controlling the drive apparatus, wherein the main body part is configured such that a mount surface for the operating means is set at a tilt angle tilted toward the grasping portion by a predetermined angle.

With the configuration, by setting the mount surface for the operating means of the main body part at the tilt angle tilted toward the grasping portion by the predetermined angle, it is made easy for the finger of the hand of the operator grasping the grasping portion to reach the operating means at an operation time of the bending portion of the endoscope, which results in easiness of the operation of the operating means.

Preferably, the tilt angle is set such that an angle defined by a flat surface of the mount surface for the operating means and the center line of the grasping portion is an angle smaller than 90°.

With the configuration, by setting the tilt angle at which the mount surface for the operating means of the main body part is tilted toward the grasping portion such that an angle formed by a plane of the mount surface for the operating means and the center line of the grasping portion is less than 90°, it is made easy for the finger of the hand of the operator grasping the grasping portion to operate the operating means at an operation time of the bending portion of the endoscope.

Preferably, the grasping portion includes a flat-shaped first receiving portion receiving a root portion of the thumb of the operator and a second receiving portion disposed on a side surface opposite to the first receiving portion and receiving a finger of the operator other than the thumb, and the main body part has a first mount surface arranged on a side surface positioned on the side of the first receiving portion and a second mount surface arranged on a side surface positioned on the side of the second receiving portion.

With the configuration, since a thumb of a hand of an operator is received by the flat-shaped first receiving portion of the grasping portion, a finger of the hand other than the thumb is received by the second receiving portion arranged on a side surface opposite to the first receiving portion, the first mount surface of the main body part is disposed on a side surface positioned on the side of the first receiving portion, and the second mount surface is disposed on a side surface positioned on the side of the second receiving portion, it is made easy for the finger of the hand of the operator to reach the operating means of the main body part at an operation time of the bending portion of the endoscope, which results in easiness of the operation.

Preferably, one of a trackball and an operation button indicating a bending direction is disposed on the first mount surface, and the second mount surface includes a suction switch controlling suction action of the endoscope, a gas-feed and water-feed switch controlling gas-feed and water-feed actions of the endoscope, and a video switch of the endoscope are arranged on the second mount surface.

With the configuration, by arranging either one of the trackball or the operation button indicating the bending direction on the first mount surface of the main body part, it is made easy for the finger of the hand of the operator grasping the grasping portion to reach the trackball and the operation button of the main body part at an operation time of the bending portion of the endoscope, which results in easiness of the operation can be provided.

According to the present invention, an operation apparatus which can be operated easily even when an operation member such as a joystick is moved in a direction far from a grasping portion and an operation apparatus for an endoscope.

### Brief Description of Drawings

FIG. 1 is a perspective view showing a schematic configuration of an overall system of a medical apparatus according to a first embodiment of the present invention;
FIG. 2 is a schematic block diagram showing the medical apparatus according to the first embodiment;
FIG. 3 is a schematic configuration diagram of a main portion showing a motorized bending endoscope of the medical apparatus according to the first embodiment;
FIG. 4 is a partially sectional side view of a joystick apparatus of a remote controller operation portion of the motorized bending endoscope according to the first embodiment;
FIG. 5 is a vertical sectional view showing a mount portion of the joystick apparatus of an operation apparatus according to the first embodiment;
FIG. 6 is a vertical sectional view of a main portion showing an internal configuration of the joystick apparatus of the operation apparatus according to the first embodiment;
FIG. 7A is a perspective view showing a finger receiving portion of the joystick apparatus of the operation apparatus according to the first embodiment;
FIG. 7B is a sectional view of the finger receiving portion, taken along VIIB-VIIB in FIG. 7A;
FIG. 8 is a vertical sectional view showing an internal configuration a gas-feed and water-feed switch of the operation apparatus according to the first embodiment;
FIG. 9 is a characteristic diagram for explaining an operation of the gas-feed and water-feed switch of the operation apparatus according to the first embodiment;
FIG. 10 is a side view showing a use state of the operation apparatus according to the first embodiment;
FIG. 11 is a side view showing an operation apparatus according to a second embodiment of the present invention;
FIG. 12 is a side view showing an operation apparatus according to a third embodiment of the present invention; and
FIG. 13 is a front view showing a bending operation button of the operation apparatus according to the third embodiment.

### Best Mode for Carrying Out the Invention

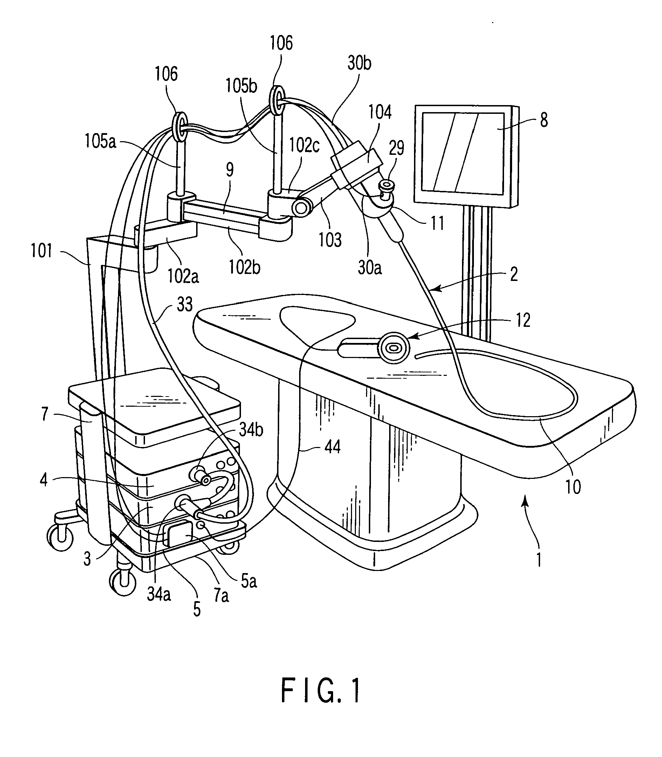

A first embodiment of the present invention will be explained below with reference to FIG. 1 to FIG. 10. FIG. 1 shows a schematic configuration of an overall system of a medical apparatus. In the medical apparatus, a cart 7 carrying a system for a motorized bending endoscope 2 is disposed at a position near a patient bed 1. A motorized bending endoscope apparatus which is a medical apparatus mainly includes the motorized bending endoscope 2, a light source apparatus 3, a video processor 4, a pump unit 5, and system power source 6.

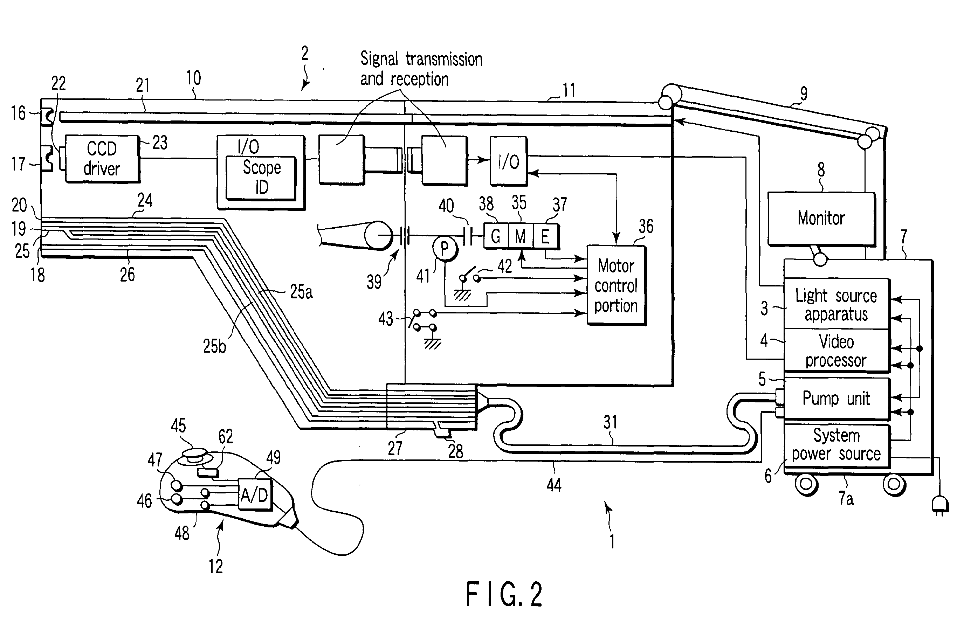

As shown in FIG. 2, the light source apparatus 3, the video processor 4, the pump unit 5, and the system power source 6 arranged in a stacking manner are accommodated in the cart 7 on a carriage 7a provided with, for example, casters or the like so as to be movable on a floor.

Further, the cart 7 is provided with a monitor 8 and has an endoscope arm 9. The monitor 8 receives video signals from the video processor 4 to display predetermined endoscope images. The endoscope arm 9 comprises a plurality of movable arm portions holding the motorized bending endoscope 2 movably, and the like. The monitor 8 is provided on a side portion of the patient bed 1 in a standing state.

The endoscope arm 9 includes an approximately L-shaped supporting arm 101 provided on the cart 7 in a standing manner, a plurality of, three (first to third) in the embodiment, horizontally moving arms 102a, 102b, and 102c, and a vertically moving arm 103. One end portion of the first horizontally moving arm 102a is coupled to an upper end portion of the supporting arm 101 so as to be rotationally movable about a rotationally moving shaft provided so as to extend in a vertical direction. One end portion of the second horizontally moving arm 102b is coupled to the other end portion of the first horizontally moving arm 102a so as to be rotationally movable about a rotationally moving shaft provided so as to extend in a vertical direction. One end portion of the third horizontally moving arm 102c is coupled to the other end portion of the second horizontally moving arm 102b so as to be rotationally movable about a rotationally moving shaft provided so as to extend in a vertical direction. A proximal end of the vertically moving arm 103 is coupled to the other end portion of the third horizontally moving arm 102c so as to be rotationally movable about a rotationally moving shaft provided so as to extend in a horizontal direction. Further, an endoscope holding portion 104 is disposed at a distal end portion of the vertically moving arm 103.

A first supporting rod 105a provided so as to extend in a vertical direction is coupled to a coupling portion between the first horizontally moving arm 102a and the second horizontally moving arm 102b. A second supporting rod 105b provided so as to extend in a vertical direction is coupled to a coupling portion between the second horizontally moving arm 102b and the third horizontally moving arm 102c. Ring-like bundle retainers 106 are fixed to upper end portions of the first supporting rod 105a and the second supporting rod 105b, respectively.

The motorized bending endoscope 2 mainly comprises an elongated insertion portion 10, a motorized bending control unit 11, and a remote controller operation portion (an operation apparatus for an endoscope) 12 of the present embodiment. The insertion portion 10 is inserted into a body cavity. The motorized bending control unit 11 is coupled to a proximal end of the insertion portion 10 in an attachable and detachable manner. The remote controller operation portion is provided separately from the motorized bending control unit 11.

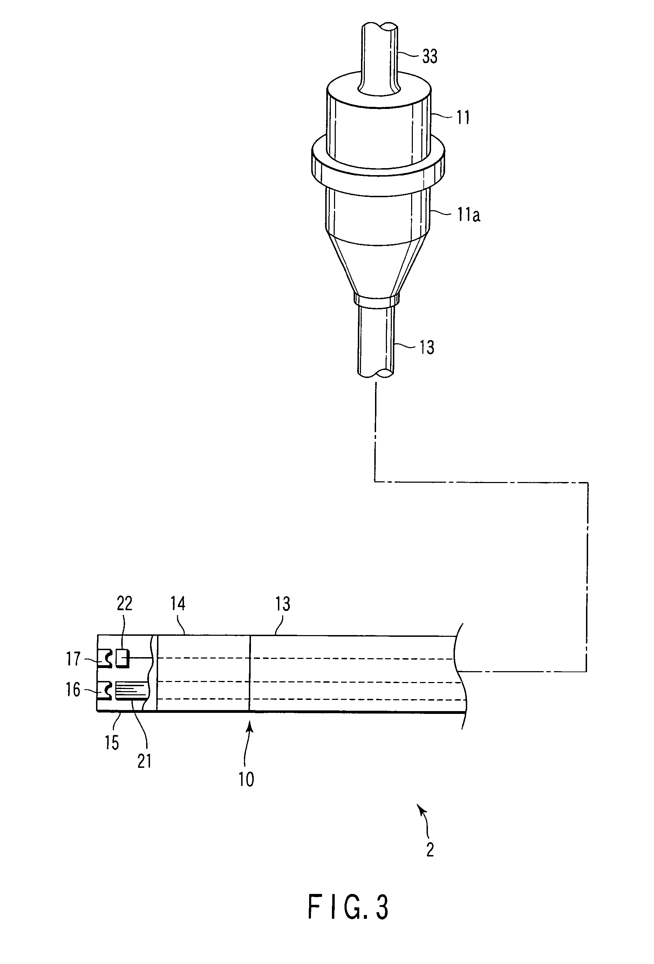

As shown in FIG. 3, the motorized bending control unit 11 has an approximately cylindrical or an approximately column-shaped unit main body 11a. One end portion of the insertion portion 10 is attachably and detachably coupled to one end portion of the unit main body 11a, and each of one end portions of a universal cable 33 and a fluid tube 31 which have flexibility, described later is coupled to the other end portion of the unit main body 11a. The unit main body 11a of the motorized bending control unit 11 is attached to an endoscope holding portion 104 of the endoscope arm 9. Thereby, the endoscope 2 is supported by the endoscope arm 9 to be movable within a predetermined range.

Further, the universal cable 33 and the fluid tube 31 are inserted into the respective bundle retainers 106 of the first supporting rod 105a and the second supporting rod 105b movably in their axial directions, respectively and rotational movably/rotatably about their axes.

As shown in FIG. 3, the insertion portion 10 includes an elongated flexible tube portion 13, a bending portion 14 whose proximal end portion is coupled to a distal end of the flexible tube portion 13, and a distal end hard portion 15 whose proximal end portion is coupled to a distal end of the bending portion 14. An illumination lens 16 of an illumination optical system, an observation lens 17 of an observation optical system, a distal end opening portion 18 of a treatment tool insertion channel shown in FIG. 2, a gas-feed and water-feed nozzle 19, a forward water-feed opening portion 20, and the like are arranged on a distal end face of the distal end hard portion 15.

Further, a distal end portion of a light guide fiber 21 guiding illumination light is disposed behind the illumination lens 16. An image pickup element such as CCD 22 for photoelectrically converting an image focused by the observation lens 17 and a CCD driver 23 driving the CCD 22 are disposed behind the observation lens 17.

Further, a forward water-feed conduit 24, a gas-feed conduit 25a, a water-feed conduit 25b, a treatment tool insertion conduit 26 also serving as a suction conduit are provided in the insertion portion 10. A distal end portion of the forward water-feed conduit 24 is coupled to the forward water-feed opening portion 20. A distal end portion of the gas-feed conduit 25a is coupled to a distal end portion of the water-feed conduit 25b. A gas-feed and water-feed conduit 25 is formed ahead of a coupling portion between the water-feed conduit 25b and the gas-feed conduit 25a. A distal end portion of the gas-feed and water-feed conduit 25 is coupled to a gas-feed and water-feed nozzle 19. A distal end portion of the treatment tool insertion conduit 26 is coupled to the distal end opening portion 18.

A tube connector 27 is provided at a proximal end of the insertion portion 10. A treatment tool insertion portion 28 coupled to a proximal end of the treatment tool insertion conduit 26 is provided in the tube connector 27, and respective proximal ends of the forward water-feed conduit 24, the gas-feed conduit 25a, and the water-feed conduit 25b are coupled to the tube connector 27, respectively. A forceps plug 29 attached with a suction tube is attached to the treatment tool insertion portion 28. A treatment tool such as forceps inserted from the treatment tool insertion portion 28 via the forceps plug 29 with a suction tube can be protruded from the distal end opening portion 18 on a front face of the insertion portion 10 on the distal end side thereof through the treatment tool insertion conduit 26.

Incidentally, one end portion of a suction tube 30a prepared as a separate member is coupled to the forceps plug 29 with a suction tube. The treatment tool insertion conduit 26 is coupled to the suction tube 30a prepared as a separate member via the forceps plug 29 with a suction tube of the treatment tool insertion portion 28. Thereby, the treatment tool insertion conduit 26 is also used as a conduit for sucked material obtained at a suction time. The sucked material can be sucked from the treatment tool insertion conduit 26 to the suction tube 30a via the forceps plug 29 for a suction tube of the treatment tool insertion portion 28.

One end portion of a gas-feed and water-feed tube 30b is coupled to the tube connector 27. A fluid tube 31 comprises the gas-feed and water-feed tube 30b and the suction tube 30a. The other end portion of the fluid tube 31 is coupled to the pump unit 5. Thereby, the pump unit 5 communicates with the distal end of the insertion portion 10 via the forward water-feed conduit 24, the gas-feed conduit 25a, the water-feed conduit 25b, the gas-feed and water-feed conduit 25, the treatment tool insertion conduit 26, or the suction tube 30a and the gas-feed and water-feed tube 30b of the fluid tube 31. When pump unit 5 is driven so that gas-feed or water-feed and suction actions are performed, gas-feed or water-feed and suction from the distal end face of the insertion portion 10 can be performed.

A fluid control cassette 5a is attachably and detachably attached to the pump unit 5. The fluid control cassette 5a is provided with a flow rate adjusting mechanism having valves regarding gas-feed, water-feed, and suction. The pump unit 5 drives the flow rate adjusting mechanism of the fluid control cassette 5a.

The bending portion 14 of the motorized bending endoscope 2 is configured by arranging a plurality of approximately ring-like bending pieces in parallel along the axial direction of the insertion portion 10 and connecting them via rotational moving pins such as rivets in a rotationally movable manner, respectively. Further, distal ends of four wires for bending operation for operating the bending portion 14 in a bending manner, for example, in four upward, downward, leftward, and rightward directions are connected to the bending portion 14. A proximal end of each wire extends toward on the side of the proximal end of the insertion portion 10. The wires receive driving force from the motorized bending control unit 11 to be driven in a pulled manner. Thereby, the bending portion 14 can bend from a normal straight state (non-bent state) where it extends straightly so that the bent angle is 0° to a bent shape where it has been operated to an arbitrary angle in a bending manner in an upward, downward, leftward, or rightward direction.

The light guide fiber 21 and a signal cable for the CCD 22 are arranged in the universal cable 33. A light connector portion 34a connected to the light source apparatus 3 is coupled to a distal end portion of the universal cable 33. An electric connector portion 34b is coupled to a side portion of the light connector portion 34a via an electric cable. The electric connector portion 34b is connected to the video processor 4.

Further, the light guide fiber 21 extends through the motorized bending control unit 11 and the insertion portion 10 up to the distal end portion of the insertion portion 10. In this case, the light guide fiber 21 is coupled to an attaching and detaching site between the motorized bending control unit 11 and the insertion portion 10 via a light connection connector (not shown) and it communicates therewith in a state that the motorized bending control unit 11 and the insertion portion 10 have been engaged with each other. Thereby, illumination light from the light source apparatus 3 is transmitted to the side of the distal end portion of the insertion portion 10 via the light guide fiber 21 to be emitted forward from the illumination lens 16 so that a subject is illuminated.

The signal cable transmitting a video signal from the CCD 22 inserts within the insertion portion 10 and the motorized bending control unit 11 to be connected to a predetermined terminal of the video processor 4 by an electric connector portion 34b through inside of the universal cable 33. In this case, the signal cable is coupled to the predetermined terminal at an attachable and detachable site between the motorized bending control unit 11 and the insertion portion 10 detachably through a connection connector (not shown) or the like, and the signal cable and the predetermined terminal are electrically connected in a state that both the motorized bending control unit 11 and the insertion portion 10 have been engaged with each other.

An endoscope image is focused on the CCD 22 by the observation lens 17 and it is converted to a video signal by the CCD 22. The video signal output from the CCD 22 is input into the video processor 4. The monitor 8 is electrically connected to the video processor 4. Thereby, a video signal output from the video processor 4 after subjected to a predetermined signal processing in the video processor 4 is transmitted to the monitor 8. The monitor 8 receives this video signal so that the endoscope image is displayed on a display screen of the monitor 8.

The motorized bending control unit 11 comprises a electric motor (bending drive means) 35 which is a drive source electrically driving the bending portion 14, a motor control portion 36 performing overall control of the motorized bending control unit 11 including the electric motor 35, an encoder 37 changing operation state such as a rotational speed or a rotational amount of the electric motor 35 to data, a reduction gear mechanism 38 reducing rotation power of the electric motor 35, an electromagnetic clutch 40 coupled to the reduction gear mechanism 38 to transmit the rotation power of the electric motor 35 to a power coupling portion 39 on the side of the insertion portion 10, a potentiometer 41 serving as rotation position detecting means, a clutch action detecting switch 42 detecting action of the electromagnetic clutch 40, an attached and detached state detecting switch 43 detecting an engagement state between the insertion portion 10 and the motorized bending control unit 11, and the like.

The system power source 6 is electrically connected to each of the light source apparatus 3, the video processor 4, the pump unit 5, and the monitor 8. An electric cable 44 extending from the remote controller operation portion 12 is connected to the pump unit 5.

The remote controller operation portion 12 comprises various operation members such as a joystick apparatus (bending operation input means) 45 which is a bending operation apparatus instructing and inputting bending action to the bending portion 14, a gas-feed and water-feed switch 46 issuing a gas-feed and water-feed operation instruction, and a suction switch 47 issuing a suction operation instruction, various video switches 48 remotely operating various functions of the videos processor 4, such as video shooting, an AD converter 49 electrically connected to these operation input means, and the like. Incidentally, the AD converter 49 receives electric signals occurring from the various video switches 48 for operating video shooting and the like to perform AD conversion processing for producing predetermined operation instruction signals. Further, the plurality of video switches 48 on the remote controller operation portion 12 can be each allocated with switch functions such as freeze (still image) instruction, or release, for example. By performing switch operation of the video switch 48, signal processing corresponding to the allocated function, for example, an action outputting a still image to the display screen of the monitor 8 is performed.

According to various instruction signals occurring due to operations to respective operation members on the remote controller operation portion 12, control signals for performing controls corresponding to proper instruction signals from the AD converter 49 are output toward various apparatuses. Thereby, drive control of the motorized bending control unit 11 is performed and overall control on the light source apparatus 3, the video processor 4, the pump unit 5, and the like is performed.

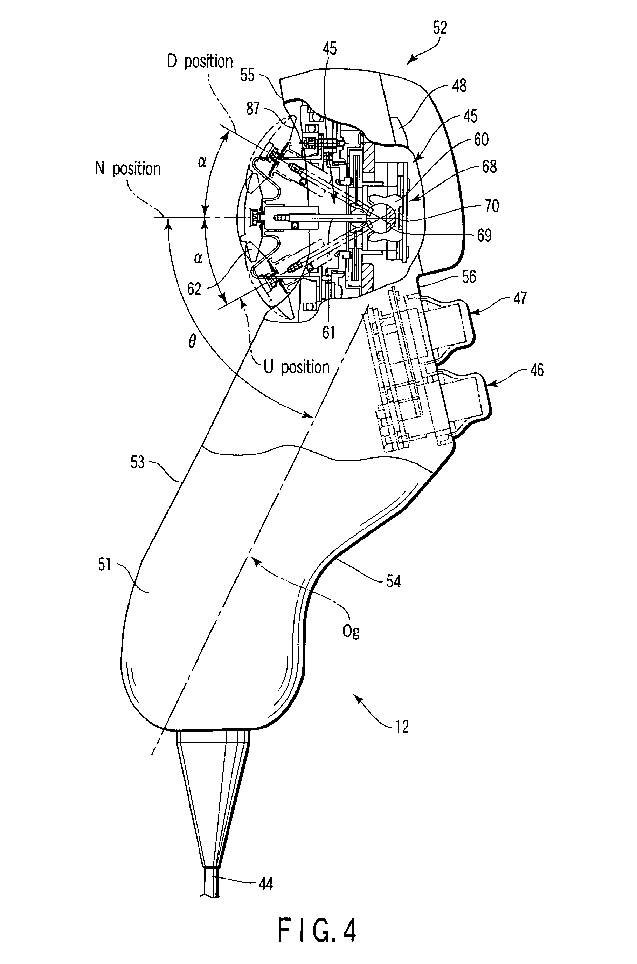

FIG. 4 shows a specific configuration of the remote controller operation portion 12 serving as the operation apparatus of the endoscope according to the embodiment. The remote controller operation portion 12 includes a grip portion (grasping portion) 51 which can be grasped by an operator, and a main body part 52 arranged on the side of an upper end portion of the grip portion 51. The joystick apparatus 45, the gas-feed and water-feed switch 46, the suction switch 47, and the plurality of video switches 48 are arranged on the main body part 52. An electric cable 44 is connected to the side of a lower end portion of the grip portion 51.

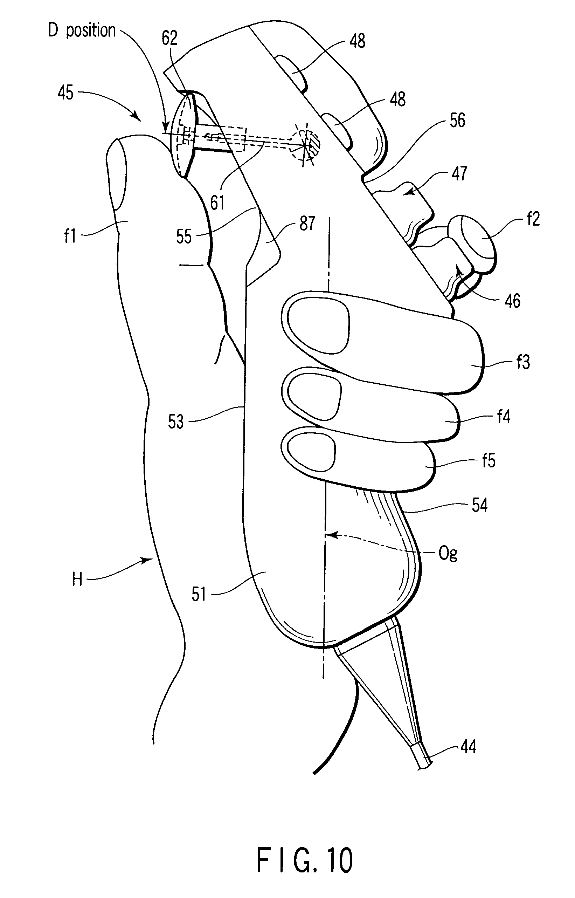

As shown in FIG. 10, the grip portion 51 includes a flat-shaped first receiving portion 53 receiving a root portion of a thumb f1 of a left hand H of an operator, and a second receiving portion 54 disposed on a side surface opposite to the first receiving portion 53 to receive fingers other than the thumb f1, for example, a long finger f3, an annular finger f4, and a little finger f5. Incidentally, such a configuration is adopted that an operator can move an index finger f2 freely.

Further, the first receiving portion 53 is formed by a flat surface approximately parallel to a grip axis Og which is the center line of the grip portion 51. The second receiving portion 54 is formed a curved shape where an approximately central portion in an axial direction is recessed to a flat surface approximately parallel to the grip axis Og. When an operator grasps the grip portion 51 of the remote controller operation portion 12 with his/her left hand H, a root portion of his/her thumb f1 is received by the flat-shaped first receiving portion 53 of the grip portion 51 and his/her long finger f3, annular finger f4, and little finger f5 are received by the second receiving portion 54 arranged on the side surface opposite to the first receiving portion 53. In a state that the operator has grasped the grip portion 51 of the remote controller operation portion 12 with his/her left hand H in this manner, a stick shaft 61 (described later) of the joystick apparatus 45 of the remote controller operation portion 12 is operated by his/her thumb f1 of the left hand H grasping the grasping portion 51 and either one of the gas-feed and water-feed switch 46 and the suction switch 47 is selectively operated by his/her index finger f2.

A first mount surface 55 on which the joystick apparatus 45 is mounted and a second mount surface 56 arranged on a side surface opposite to the first mount surface 55 are provided on the main body part 52. The gas-feed and water-feed switch 46, the suction switch 47, and the plurality of video switches 48 which are operating means other than the joystick apparatus 45 are arranged on the second mount surface 56.

Further, the main body part 52 is formed in a bending manner in a state that it has been tilted obliquely upwardly in a direction of the grip axis Og which is the center line of the grip portion 51. Thereby, the first mount surface 55 and the second mount surface 56 of the main body part 52 are set to a tilt angle θ tilted toward the grip portion 51 by a predetermined angle. The tilt angle θ is set such that angles defined by planes of the first mount surface 55 and the second mount surface 56 of the main body part 52, and the grip axis Og which is the center line of the grip portion 51 are angles smaller than 90°.

The first mount surface 55 of the main body part 52 is disposed on a side surface positioned on the side of the first receiving portion 53 of the grip portion 51. The second mount surface 56 is disposed on a side surface positioned on the side of the second receiving portion 54 of the grip portion 51.

Further, the gas-feed and water-feed switch 46 is disposed on the second mount surface 56 near a position nearest the second receiving portion 54 and the suction switch 47 is disposed at a position far from the second receiving portion 54 beyond the gas-feed and water-feed switch 46 in parallel with the gas-feed and water-feed switch 46. The plurality of video switches 48 is disposed at a position further far from the second receiving portion 54 beyond the suction switch 47 in parallel therewith.

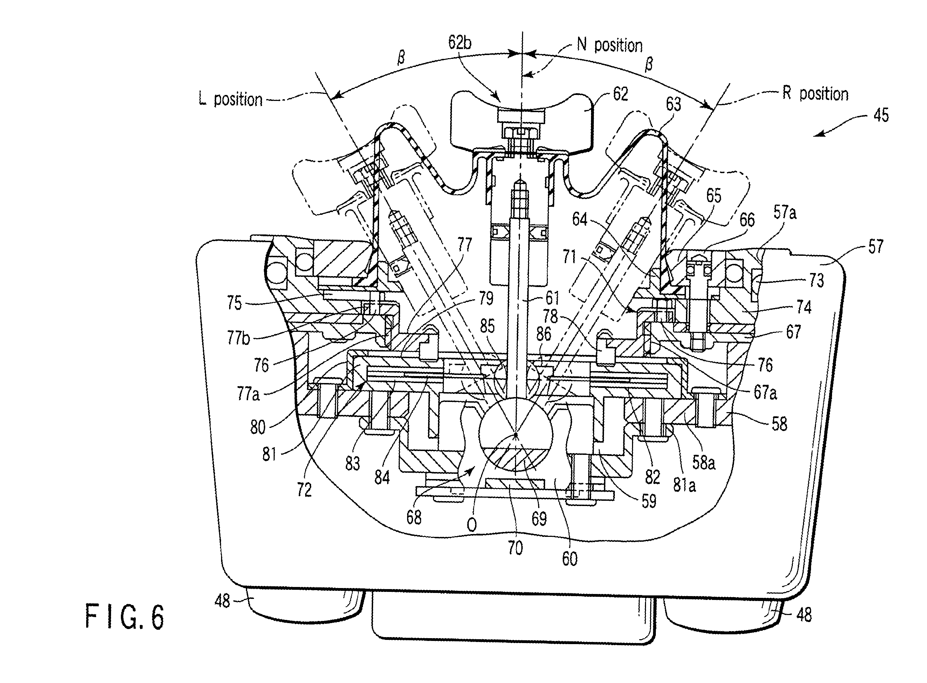

FIG. 6 shows an internal configuration of the joystick apparatus 45. In the joystick apparatus 45, an approximately cylindrical main frame 58 is disposed within an approximately column-like shape recessed portion 57a provided in a casing 57 on the side of the first mount surface 55 of the main body part 52 of the remote controller operation portion 12. A flat-like extending portion 58a provided so as to extend inwardly is formed in a lower end portion of the main frame 58. A cylindrical joystick main body fixing member 59 with a bottom is attached to a lower surface of the lower end portion extending portion 58a of the main frame 58. A joystick main body 60 is arranged on a bottom face of the joystick main body fixing member 59. The joystick main body 60 is attached to the main frame 58 via the joystick main body fixing member 59.

Further, the stick shaft 61 which can be operated by an operator is disposed on an upper surface of the joystick main body 60 in a standing manner. A proximal end of the stick shaft 61 is rotatably pivoted about a rotational movement supporting axis O within the joystick main body 60.

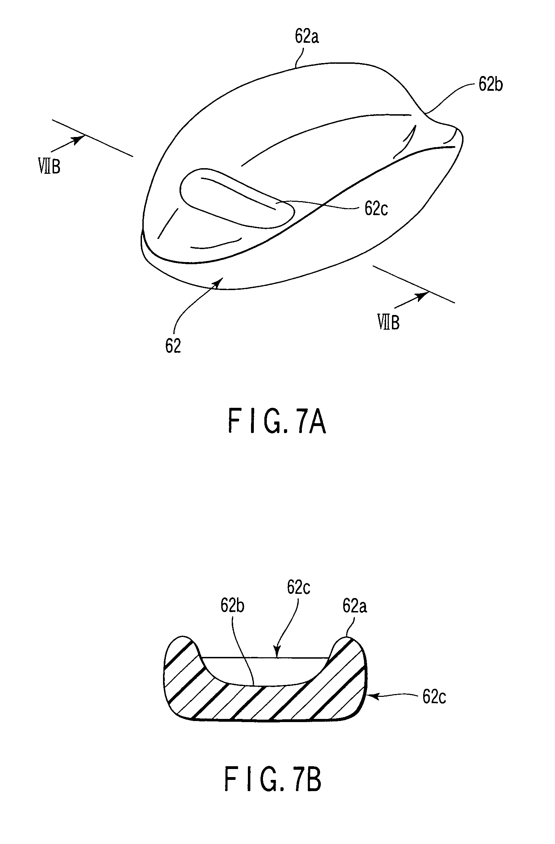

Further, a finger receiving portion 62 which is a key top is disposed at a head portion of the stick shaft 61. As shown in FIG. 7A, the finger receiving portion 62 is formed with arc-shaped projecting portions 62a extending along a direction corresponding to upward and downward bending directions of the bending portion 14 and an arc-shaped recessed portion 62b extending along leftward and rightward bending directions of the bending portion 14, respectively.

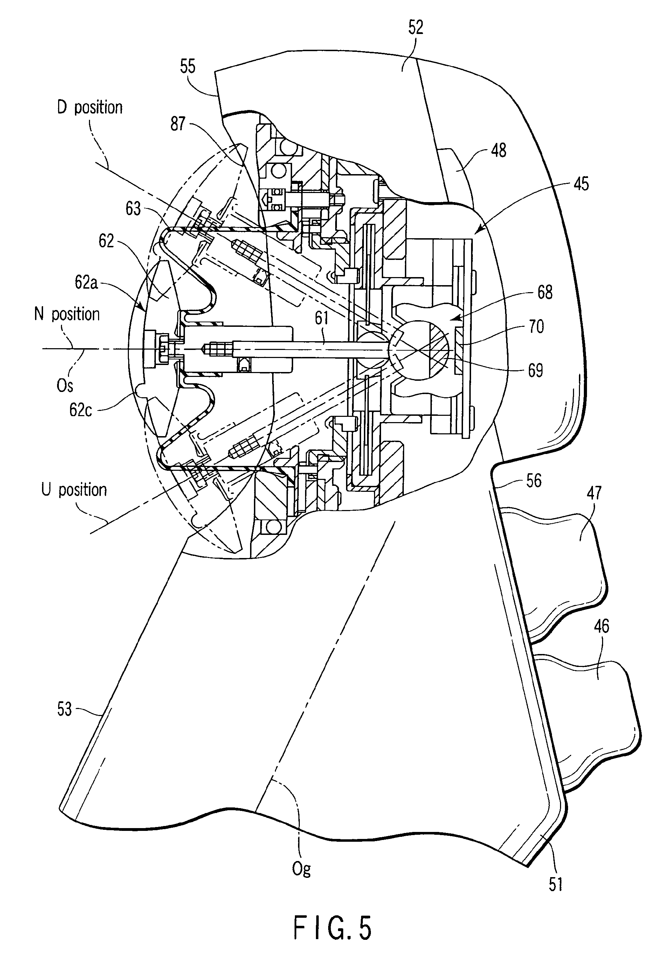

A rubber cover 63 is disposed on an upper portion of the stick shaft 61. A peripheral edge portion of the rubber cover 63 is fixed to an engagement mechanism frame 67 described later by screws 66 in a state that it is stacked on an approximately ring-like restricting member 64 restricting an tilting range of the stick shaft 61 and the peripheral edge portion is brought in pressure-contact with the restricting member 64 such that it is sandwiched between the restricting member 64 and an approximately ring-like pressing member 65. An O-ring for sealing is attached to the screws 66. Thereby, the stick shaft 61 is covered with the rubber cover 63, thereby achieving a waterproof structure. By operating the finger receiving portion 62 of the head portion of the joystick apparatus 45, the stick shaft 61 is tilted in a state that it can be rotationally moved about the rotational movement supporting point O and it issues instruction input of a bending operation according to the tilting. Incidentally, a solid line in FIG. 6 shows a state that the stick shaft 61 of the joystick apparatus 45 has been held at a neutral position (N position), while imaginary lines in FIG. 6 show an L position (leftward tilted position) and a R position (rightward tilted position) where the stick shaft 61 of the joystick apparatus 45 has been tilted to leftward and rightward maximum tilt angles β. A solid line in FIG. 4 and FIG. 5 shows a state that the stick shaft 61 of the joystick apparatus 45 has been held at the neutral position (N position), while imaginary lines in FIG. 4 and FIG. 5 shows a U position (upwardly tilted position) and a D position (downwardly tilted position) where the stick shaft 61 of the joystick apparatus 45 has been tilted to upward and downward maximum tilt angles α.

A detecting apparatus (operation amount detecting means) 68 of a tilt direction and a tilt angle is disposed within the joystick main body 60. The detecting apparatus 68 includes a magnet (action member) 69 disposed at a lower end portion of the stick shaft 61 and a Hall element (output portion) 70 disposed so as to face the magnet 69 below the magnet 69 in a spaced manner. The Hall element 70 is connected to the motor control portion 36 of the motorized bending control unit 11 via an electric cable 44 and through the pump unit 5. Change of electric field of the magnet 69 is detected by the Hall element 70 at a tilt time of the stick shaft 61. At this time, the electric field of the magnet 69 is converted to a voltage by the Hall element 70 to be output to the motor control portion 36 of the motorized bending control unit 11, so that the tilt direction and the tilt angle of the stick shaft 61 are detected.

The joystick apparatus 45 according to the embodiment includes a brake mechanism (braking means) 71 serving as bending angle fixing means and operation feeling imparting means 72 imparting proper operation feeling at a tilt operation time of the stick shaft 61.

The ring-like engagement mechanism frame 67 screw-fixed on the main frame 58 is provided in the brake mechanism 71. An O-ring 73 performing sealing between an upper end portion outer peripheral face of the engagement mechanism frame 67 and an inner peripheral face of the recessed portion 57a of the casing 57 of the remote controller operation portion 12 is provided on the upper end portion outer peripheral face.

An engagement ring 74 is disposed on the engagement mechanism frame 67. The engagement ring 74 is supported on the engagement mechanism frame 67 so s to be rotationally movable about the center line of the joystick apparatus 45 in a spinning direction. A brake operation lever (bake operation portion) (not shown) is provided on the engagement ring 74 to project upwardly between an upper end portion of the engagement mechanism frame 67 and the pressing member 65. Further, a relay ring 75 is fixed on an inner end portion of the engagement ring 74. A plurality of pins 76 is provided on the relay ring 75 so as to extend downwardly.

A female screw portion 67a is formed on an inner peripheral face of the engagement mechanism frame 67. A male screw portion 77a formed on an outer peripheral face of a ring-like brake-mount member 77 is screwed into the female screw portion 67a. A projecting portion projecting toward the stick shaft 61 positioned inside is provided on a lower end portion of the brake-mount member 77, and a brake member (abutting member) 78 formed of a member with a large friction coefficient such as rubber is attached to the projecting portion.

Further, insertion holes 77b into which the pins 76 of the relay ring 75 are inserted movably in the axial directions are formed in an upper end portion of the brake-mount member 77. When the engagement ring 74 is operated to the engagement mechanism frame 67 in a rotationally moving manner by the brake operation lever, rotation of the engagement ring 74 is transmitted to the brake-mount member 77 via the pins 76 of the relay ring 75. Thereby, the brake-mount member 77 is rotated together with the engagement ring 74. At this time, the brake-mount member 77 is moved in the axial direction of the stick shaft 61 (moved vertically) in a state that the male screw portion 77a of the brake-mount member 77 moves in a screwing manner along the female screw portion 67a of the engagement mechanism frame 67 according to the rotation of the brake-mount member 77. Thereby, switching operation between a state that the brake member 78 has been separated from a brake plate 79 described later (non-braking state) and a state that the brake member 78 has been brought in pressure contact with the brake plate 79 (braked state) is performed.

When the engagement ring 74 is operated in a brake operation direction in a rotationally moving manner by the brake operation lever in a state that the stick shaft 61 has been tilted to an arbitrary angle, the brake member 78 of the brake-mount member 77 is brought in pressure contact with the brake plate 79. At this time, the stick shaft 61 is fixed (locked) at the tilt angle state due to frictional force occurring when the brake member 78 has been brought in pressure contact with the brake plate 79 even if the operator releases his/her hand from the stick shaft 61.

A damper case fixing member 80 is attached to an upper surface of the lower end portion extending portion 58a of the main frame 58. A damper case (resistance body holding means) 81 disposed between the damper case fixing member 80 and the lower end portion extending portion 58a of the main frame 58 is provided on the operation feeling imparting means 72. A ring-like receiving recessed portion 82 is formed in the damper case 81 so as to extend over an entire inner peripheral face of a ring-like case main body 81a thereof. A viscosity fluid (resistance material) 83 and a disk-like moving member 84 are received in the receiving recessed portion 82. The viscosity fluid 83 is received in the receiving recessed portion 82 in a sealed manner. An outer peripheral portion of the moving member 84 is inserted into the viscosity fluid 83 sealed in the receiving recessed portion 82 of the damper case 81 in a putting-in state thereof. Further, the brake plate 79 is fixed on an upper surface of the damper case 81.

A spherical coupling member 85 engaged with an intermediate portion of the stick shaft 61 and a fixing member 86 for the spherical coupling member 85 are provided in the axial center portion of the moving member 84. A fitting hole portion through which the stick shaft 61 is fitted movably in advancing and retreating direction is formed in the axial center portion of the spherical coupling member 85. A outer end portion of the fixing member 86 is fitted and fixed in an inner peripheral surface of the moving member 84. Further, a spherical bearing portion coming in sliding contact with a spherical surface of the spherical coupling member 85 is formed on an inner end portion of the fixing member 86. Thereby, pressing force from the stick member 61 is transmitted to the moving member 84 within the damper case 81 via the spherical coupling member 85 and the fixing member 86 at a tilt action time of the stick shaft 61 so that the moving member 84 is laterally moved within the damper case 81 together with the tilting action of the stick shaft 61. Sliding resistance of the moving member 84 is increased by the viscosity fluid 83 sealed in the receiving recessed portion 82 of the damper case 81 at a moving time of the moving member 84.

The joystick apparatus 45 of the embodiment is mounted on the first mount surface 55 of the main body part 52 of the remote controller operation portion 12 in the following manner. That is, as shown in FIG. 4, the neutral position (N position) of the stick shaft 61 of the joystick apparatus 45 is set to the tilt angle θ tilted toward the grip portion 51 by the predetermined angle. The tilt angle θ is set such that an angle formed between the center line Os of the stick shaft 61 and the center line Og of the grip portion 51 is an angle smaller than 90° at the neutral position of the stick shaft 61. It is preferable that the tilt angle is in a range from about 60° to 70°±10°.

Further, a recess-shaped depression portion 87 is provided at a mount portion of the joystick apparatus 45 on the first mount face 55 of the main body part 52 of the remote controller operation portion 12. The rotational movement supporting point O of the stick shaft 61 of the joystick apparatus 45 is disposed at an inner bottom portion position of the depression portion 87.

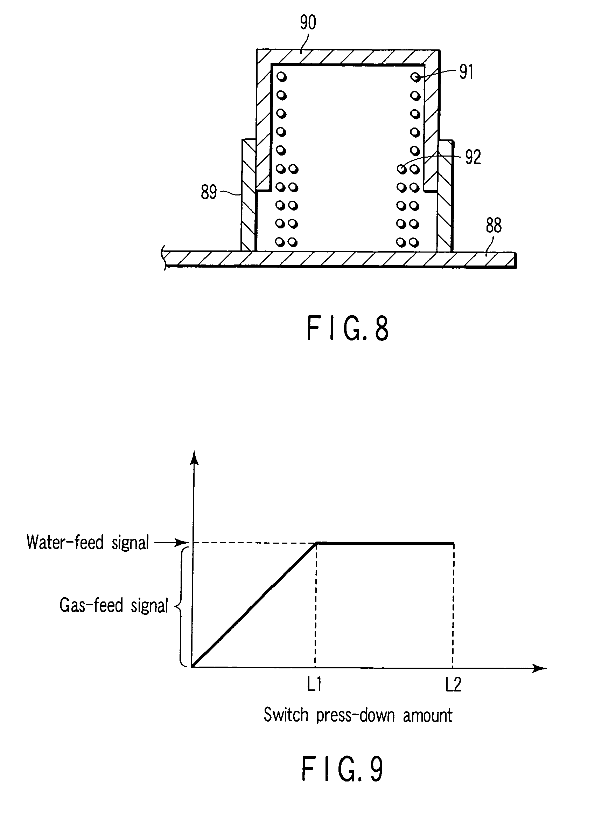

The gas-feed and water-feed switch 46 disposed on the second mount surface 56 of the main body part 52 of the remote controller operation portion 12 comprises a two-stage switch, and the suction switch 47 comprises a one-stage switch. FIG. 8 is an explanatory diagram for explaining a principle of an operation of the gas-feed and water-feed switch 46. In FIG. 8, reference numeral 88 is a switch substrate. A cylindrical fixed cylinder 89 and a press-down member 90 sliding in upward and downward directions relative to the fixed cylinder 89 are provided on the switch substrate 88.

Further, two (first and second) helical compressing springs 91 and 92 are arranged within the fixed cylinder 89. The first helical compressing spring 91 is set to be longer in length in the sliding direction of the press-down member 90 than the second helical compressing spring 92. The press-down member 90 is normally held at a home position (non-operated position) by spring force of the first helical compressing spring 91.

The press-down member 90 is operated against only spring force of the first helical compressing spring 91 in a pressed manner in an initial stage of a press-down operation of the press-down member 90. The second helical compressing spring 92 abuts on the press-down member 90 at a time point at which the press-down member 90 has been pressed down to a position of a predetermined stroke L1. Therefore, the press-down member 90 is operated in a press-down manner against spring force of spring force of the first helical compressing spring 91 plus spring force of the second helical compressing spring 92 in a range from the stroke L1 to a stroke L2 subsequent to the position of the stroke L1 at which the second helical compressing spring 92 has abuts on the press-down spring 90.

FIG. 9 is a characteristic diagram showing a relationship between a press-down amount of the press-down member 90 of the gas-feed and water-feed switch 46 and an output signal output from the gas-feed and water-feed switch 46. Here, in the gas-feed and water-feed switch 46, gas-feed signal is output in a range until the press-down amount of the press-down member 90 reaches the position of the stroke L1. Water-feed signal is output when the press-down amount of the press-down member 90 falls in the range from stroke L1 to L2.

The suction switch 47 includes a cylindrical fixed cylinder (not shown) and a press-down member sliding in upward and downward directions relative to the fixed cylinder. Only one helical compressing spring is disposed within the fixed cylinder. When the press-down member of the suction switch 47 is operated in a press-down manner, a suction signal is output.

Next, an operation of the abovementioned configuration will be explained. As shown in FIG. 10, the grip portion 51 of the remote controller operation portion 12 is grasped by a left hand H of an operator at a use time of the motorized bending endoscope apparatus according to the embodiment. At this time, when the operator grasps the grip portion 51 of the remote controller operation portion 12 with the left hand H, a root portion of his/her thumb f1 is received by the flat-like first receiving portion 53 of the grip portion 51, and the long finger f3, the annular finger f4, and the little finger f5 are received by the second receiving portion 54 disposed on the side surface opposite to the first receiving portion 53. The stick shaft 61 of the joystick apparatus 45 is operated by the thumb f1 of the left hand H grasping the grip portion 51 in a state that the operator has grasped the grip portion 51 of the remote controller operation portion 12 with the left hand H. One of the gas-feed switch 46 and the suction switch 47 is selectively operated by the index finger f2.

A bending action to the bending portion 14 is instructed and input according to operation of the joystick apparatus 45 of the remote control operation portion 12. Here, the bending portion 14 is ordinarily held in a non-bent shape corresponding to the bending angle of 0° where it extends straightly in a straight line. At this time, as shown by a solid line in FIG. 4, in the joystick apparatus 45, the stick shaft 61 is erected on the upper surface of the joystick main body 60 vertically to be held at the neutral position (N position) at which the tilt angle is 0°.

When the bending portion 14 of the motorized bending endoscope 2 is bent in an arbitrary direction, the joystick apparatus 45 of the remote controller operation portion 12 is operated. At this time, a user operates the finger receiving portion 62 at the head portion of the stick shaft 61 with the thumb f1 of his/her left hand H so as to perform operation such that the stick shaft 61 is tilted in a desired direction from the neutral position at which the tilt angle is 0°. The tilting operation of the stick shaft 61 is detected by the detecting apparatus 68 for a tilt direction and a tilt angle within the joystick main body 60. Detection signals indicating the tilt direction and the tilt angle of the stick shaft 61 detected by the detecting apparatus 68 are output to the motor control portion 36 of the motorized bending control unit 11. At this time, a tilt operation amount (a tilt direction and a tilt angle) of the stick shaft 61 of the joystick apparatus 45 from the neutral position is input into the motor control portion 36 as a bending operation input amount (bending operation instruction amount). Thereby, a control signal is output from the motor control portion 36 to the electric motor 35 so that the bending portion 14 is electrically driven. At this time, the bending portion 14 is operated in a bending manner by a bending angle corresponding to the bending operation input amount of the stick shaft 61 of the joystick apparatus 45.

When the stick shaft 61 is tilted at an operation time of the joystick apparatus 45, the moving member 84 of the operation feeling imparting means 72 moves together with the stick shaft 61. That is, pressing force is transmitted to the moving member 84 within the damper case 81 via the spherical coupling member 85 and the fixing member 86 at a tilt operation time of the stick shaft 61, so that the moving member 84 laterally moves in the same direction as the tilt direction of the stick shaft 61 within the damper case 81 according to the tilting action of the stick shaft 61.

Thus, sliding resistance of the moving member 84 is increased by the viscosity fluid 83 within the damper case 81 during motion of movement of the moving member 84 within the damper case 81. Therefore, since proper sliding resistance is always imparted to tilting operation of the stick shaft 61 during tilting action of the stick shaft 61, proper operation feeling can be obtained at an operation time of the joystick apparatus 45.

Further, when the bending portion 14 is fixed (locked) to its bent state according to operation of the joystick apparatus 45 in a state that the bending portion 14 has been operated in a bending manner by a bent angle corresponding to the bending operation input amount of the stick shaft 61 of the joystick apparatus 45, the brake operation lever (not shown) of the brake mechanism 71 is rotationally moved. At this time, the engagement ring 74 is operated to the engage mechanism frame 67 in a rotational moving manner integrally with the brake operation lever. Rotation of the engagement ring 74 is transmitted to the brake-mount member 77 via the pins 76 of the relay ring 75. Thereby, the brake-mount member 77 rotates together with the engagement ring 74. At this time, the brake-mount member 77 moves in the axial direction of the stick shaft 61 (moves vertically) in a state that the male screw portion 77a of the brake-mount member 77 moves in a screwing action along the female screw portion 67a of the engagement mechanism frame 67 according to rotation of the brake-mount member 77. Thereby, switching to a braking state can be performed by moving the brake member 78 in a direction of bringing the brake member 78 in pressure contact with the brake plate 79. At this time, the stick shaft 61 of the joystick apparatus 45 can be fixed (locked) to its tilt angle state by friction force between the brake member 78 and the brake plate 79, so that the bending portion 14 is also fixed to its bent angle state.

When the bent angle of the bending portion 14 is changed to another bent angle again, switching to non-braking state can be performed by moving the brake operation lever in the opposite direction, namely, in a direction in which the brake member 78 is separated from the brake plate 79. At this time, a frictional engagement between the brake member 78 and the brake plate 79 is released so that fixing of the bent angle can be released easily.

With the abovementioned configuration, the following effects can be achieved. That is, in the remote controller operation portion 12 according to the embodiment, the neutral position (N position) at the center in the rotational moving range of the stick shaft 61 of the joystick apparatus 45 attached to the main body part 52 to rotationally move about the rotational movement supporting point O is set to the tilt angle θ tilted toward the grip portion 51 by the predetermined angle and the joystick apparatus 45 is disposed in a tilting manner toward the side of the grip portion 51. Thereby, it is made easy for a finger of an operator to reach the stick shaft 61 of the joystick apparatus 45 even in a state that the operator has grasped the grip portion 51. For example, as shown in FIG. 10, the thumb f1 of the left hand H of the operator grasping the grip portion 51 can reach the finger receiving portion 62 of the head portion of the stick shaft 61 of the joystick apparatus 45 even in a state that the stick shaft 61 of the joystick apparatus 45 has been tilted in a direction far from the grip portion 51 to the maximum (the D position (downward tilt position) tilted to the downward maximum tilt angle α). Therefore, even if the operator moves the stick shaft 61 of the joystick apparatus 45 in the direction of the D position (the lower tilt position) away from the grip portion 51 with the thumb f1 of the left hand H of the operator grasping the grip portion 51, the operator can operate the stick shaft 61 easily.

As shown in FIG. 7A, since the finger receiving portion 62 is formed with the arc-shaped projecting portions 62a along a direction corresponding to the up-and-down bending direction of the bending portion 14, it can be made easy to confirm the upward and downward bending directions of the bending portion 14, and it is made easy for the thumb f1 of the left hand H of the operator to reach the upper surface of the finger receiving portion 62 even if the operator has tilted the stick shaft 61 of the joystick apparatus 45 in a direction far from the grip portion 51 to the maximum at an operation time of the bending portion 14 of the endoscope 2. Further, even if the operator tilts the stick shaft 61 of the joystick apparatus 45 in a direction close to the grip portion 51, the thumb f1 of the left hand H of the operator reaches the upper surface of the finger receiving portion 62 of the joystick apparatus 45.

By forming the arc-shaped recessed portion 62b along directions corresponding to the left and right bending directions of the bending portion 14, the left and right bending directions of the bending portion 14 can be confirmed easily, and the thumb f1 of the left hand H of the operator abuts on the upper ridge line of the recessed portion 62b of the finger receiving portion 62, which can result in easiness of leftward and rightward operations of the stick shaft 61 of the joystick apparatus 45.

Further, since the rib 62c for finger rest formed by protruding portions of the arc-shaped projecting portions 62a in the arc-shaped recessed portion 62b is provided on the finger receiving portion 62, the operator can cause his/her thumb f1 to catch the finger receiving portion 62 easily owing to the rib 62c for finger rest. Thereby, when the operator operates the stick shaft 61 of the joystick apparatus 45 in the upward and downward bending directions of the bending portion 14, it can be made hard for the stick shaft 61 to move in any direction other than the upward and downward bending directions of the bending portion 14, which can result in easiness of operation of the bending portion 14 in the upward and downward bending directions.

The recess-shaped depression portion 87 is provided on the first mount surface 55 of the main body part 52 of the remote controller operation portion 12, the joystick apparatus 45 is attached in the recessed portion 87, and the rotational movement supporting point O of the stick shaft 61 of the joystick apparatus 45 is disposed on the inner bottom position of the depression portion 87. Therefore, the length of the stick shaft 61 of the joystick apparatus 45 can be made long. Thereby, since the working stroke of the distal end portion of the stick shaft 61 of the joystick apparatus 45 can be elongated, operation of the stick shaft 61 of the joystick apparatus 45 can be facilitated.

The first mount surface 55 for mount the joystick apparatus 45 and the second mount surface 56 on the side surface opposite to the first mount surface 55 are provided on the main body part 52 of the remote controller operation portion 12, and the gas-feed and water-feed switch 46 and the suction switch 47 are disposed on the second mount surface 56. Therefore, in a state that an operator has grasped the grip portion 51 of the remote controller operation portion 12 with the left hand H, he/she operates the stick shaft 61 of the joystick apparatus 45 with the thumb f1 of the left hand H grasping the grip portion 51 and simultaneously can selectively operate either one of the gas-feed and water-feed switch 46 and the suction switch 47 with the index finger f2. As a result, the operator can operate the stick shaft 61 of the joystick apparatus 45 in a state that he/she is selectively operating either one of the gas-feed and water-feed switch 46 and the suction switch 47.

Since the second mount surface 56 of the main body part 52 of the remote controller operator portion 12 is tilted toward the grip portion 51 by the predetermined angle to be set to the tilt angle θ, it is made easy for the index finger f2 of the left hand H grasping the grip portion 51 to reach either one of the gas-feed and water-feed switch 46 and the suction switch 47 in a state that the operator has grasped the grip portion 51 of the remote controller operation portion 12 with his/her left hand H.

Further, the gas-feed and water-feed switch 46 which is the two-stage switch is disposed on the second mount surface 56 at a position nearest the second receiving portion 54. Therefore, switching between output of a gas-feed signal and output of a water-feed signal is made easy by adjusting the press-down amount of the press-down member 90 of the gas-feed and water-feed switch 46 in a two-stage manner.

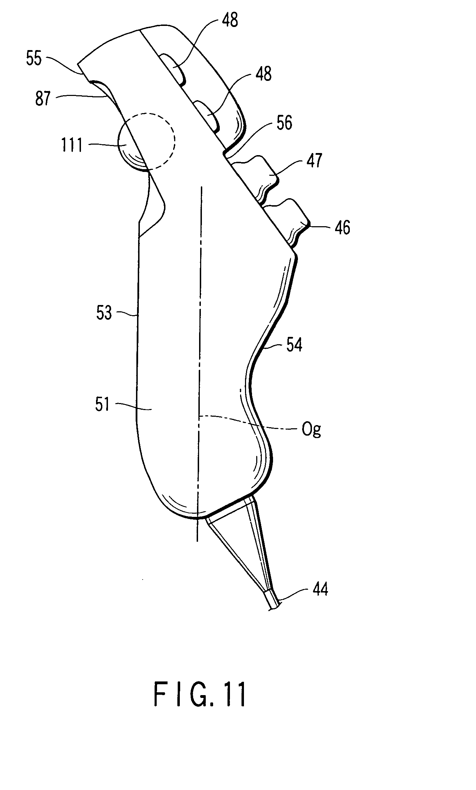

FIG. 11 shows a second embodiment of the present invention. The present embodiment has such a configuration that the configuration of the remote controller operation portion 12 according to the first embodiment (see FIG. 1 to FIG. 10) has been changed in the following manner.

That is, in the present embodiment, a trackball 111 is disposed on the first mount surface 55 of the main body part 52 of the remote controller operation portion 12 instead of the joystick apparatus 45 of the first embodiment. Incidentally, regarding the other portions, the remote controller operation portion 12 of the present embodiment has the same configuration as that of the first embodiment. In the present embodiment, same portions as those in the remote controller operation portion 12 of the first embodiment are attached with same reference numerals, and explanation thereof is omitted.

In the present embodiment, when an operator grasps the grip portion 51 of the remote controller operation portion 12 with his/her left hand H, the trackball 111 is operated by the thumb f1 of the left hand H grasping the grip portion 51 in a state that a root portion of his/her thumb f1 is received by the flat-shaped first receiving portion 53 of the grip portion 51 and his/her long finger f3, annular finger f4, and little finger f5 are received by the second receiving portion 54 arranged on the side surface opposite to the first receiving portion 53. By operating the trackball 111 in an upward direction in FIG. 11 (in a clockwise direction) in a rotational moving manner, operation of the bending portion 14 in an upward bending direction can be performed, and by operating the trackball 111 in a downward direction in FIG. 11 (in a counterclockwise direction) in a rotational moving manner, operation of the bending portion 14 in a downward bending direction can be performed. Further, by operating the trackball 111 in left and right directions in a rotational moving manner, operation of the bending portion 14 in leftward and rightward direction can be performed.

In the present embodiment, since the first mount surface 55 of the main body part 52 of the remote controller operation portion 12 is set to the tilt angle θ tilted toward the grip portion 51 by the predetermined angle, even if the operator operates the trackball 111 on the first mount surface 55 in a direction far from the grip portion 51, he/she can operate such an operation easily.

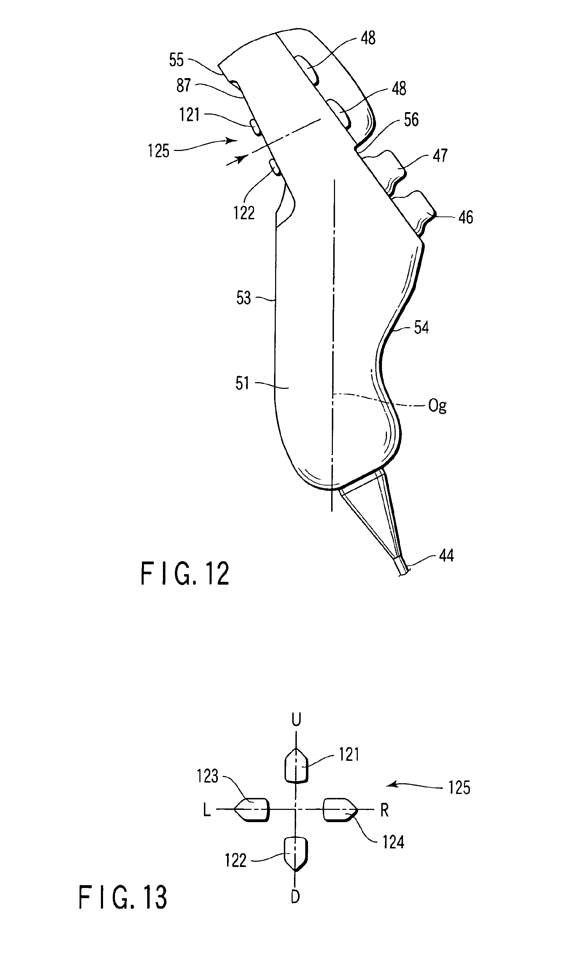

FIG. 12 and FIG. 13 show a third embodiment of the present invention. The present embodiment has such a configuration that the configuration of the remote controller operation portion 12 of the first embodiment (see FIG. 1 to FIG. 10) has been changed in the following manner.

That is, in the present embodiment, as shown in FIG. 12, a bending direction indicating apparatus 125 provided with four bending operation buttons 121 to 124 (shown in FIG. 13) indicating operation directions of four directions of upward, downward, leftward, and rightward directions is provided on the first mount surface 55 of the main body part 52 of the remote controller operation portion 12 instead of the joystick apparatus 45 of the first embodiment. Incidentally, regarding the other portions, the remote controller operation portion 12 of the present embodiment has the same configuration as that of the first embodiment. In the present embodiment, same portions as those in the remote controller operation portion 12 of the first embodiment are attached with same reference numerals, and explanation thereof is omitted.

In the present embodiment, when an operator grasps the grip portion 51 of the remote controller operation portion 12 with his/her left hand H, one of the four bending operation buttons 121 to 124 is operated by the thumb f1 of the left hand H grasping the grip portion 51 in a state that a root portion of his/her thumb f1 is received by the flat-shaped first receiving portion 53 of the grip portion 51 and his/her long finger f3, annular finger f4, and little finger f5 are received by the second receiving portion 54 arranged on the side surface opposite to the first receiving portion 53. One of the four bending operation buttons 121 to 124 is operated so that operation in one bending direction of the four directions of upward, downward, leftward, and rightward directions of the bending portion 14 can be performed.

With the abovementioned configuration, since the first mount surface 55 of the main body part 52 of the remote controller operation portion 12 is set to the tilt angle θ tilted toward the grip portion 51 by the predetermined angle, even if the operator operates the bending operation button 121 of four bending operation buttons 121 to 124 of the bending direction indicating apparatus 125 on the first mount surface 55 which is positioned in a direction far from the grip portion 51, he/she performs such an operation easily.

Further, the present invention is not limited to the abovementioned embodiments, it can be modified variously and implemented without departing from the scope of the present invention, of course.

Next, other characteristic technical items of the present application are additionally described below.
Note
(Added Claim 1) An operation apparatus comprising: a main body part having a grasping portion which can be grasped by an operator; an operation member which is disposed on the main body part while defining a position tilted toward the grasping portion by a predetermined angle as a neutral state and is supported to be capable of tilting; an action member which acts in response to tilting of the operation member; a variable resistor which changes according to action of the action member; and an output portion which outputs a resistance value of the variable resistor to an external apparatus as an instruction signal.
(Added Claim 2) A bending operation apparatus which issues an action instruction to a drive apparatus connected to a bending portion of an endoscope, comprising: a main body part having a grasping portion which can be grasped by an operator; an operation member which is disposed on the main body part while defining a position tilted toward the grasping portion by a predetermined angle as a neutral state and is supported to be capable of tilting; an action member which acts in response to tilting of the operation member; a variable resistor which changes according to action of the action member; and an output portion which outputs a resistance value of the variable resistor to an bending control apparatus controlling the drive apparatus as an instruction signal.
(Added Claim 3) A controller for a motorized bending endoscope wherein a joystick is disposed on a controller such that a stick shaft of the joystick at a neutral time of the joystick is tilted to a grip shaft in a direction of the grip portion within 90°.
(Added Claim 4) A controller for a motorized bending endoscope wherein an upper surface shape of a joystick operation member is formed such that a portion of the operation member in upward and downward operation directions is a projected approximately arc shape having the center in a stick shaft and a portion of the operation member in leftward and rightward operation directions is a recessed approximately arc shape having the center in the stick shaft.

### Industrial Applicability

The present invention is useful in a technical field using an operation apparatus operating an endoscope provided with a motorized bending portion and a technical field manufacturing an operation apparatus of the endoscope.

## Claims

1. An operation apparatus comprising:
a main body part including an operation member whose proximal end is pivoted via a rotational movement supporting point so as to be capable of rotationally moving and which is supported so as to be tiltable between a central neutral position in a rotational movement range where the operation member rotationally moves about the rotational movement supporting point and an tilt position tilted from the neutral position;
a gasping portion which can be grasped by an operator;
an action member which acts in response to tilting of the operation member; and
an output portion which outputs an instruction signal changing according to action of the action member to an external apparatus, wherein
the operation member is configured such that the neutral position is set at a tilt angle tilted toward the grasping portion by a predetermined angle.

2. The operation apparatus according to claim 1, wherein
the tilt angle is set such that an angle defined by the center line of the operation member and the center line of the grasping portion is an angle smaller than 90° at the neutral position.

3. The operation apparatus according to claim 1, wherein
the grasping portion includes a flat-shaped first receiving portion receiving a root portion of the thumb of the operator and a second receiving portion disposed on a side surface opposite to the first receiving portion and receiving a finger of a hand of the operator other than the thumb, and
the first receiving portion is disposed on the side of a mount surface for the operation member of the main body part.

4. The operation apparatus according to claim 1, wherein
the main body part is configured such that a mount surface of operating means other than the operation member is disposed on a side surface opposite to the mount surface for the operation member.

5. An operation apparatus comprising:
a main body part having operating means for operating a movable member;
a grasping portion which can be grasped by an operator;
an action member which acts in response to operation of the operating means; and
an output portion which outputs an instruction signal changing according to action of the action member to an external apparatus, wherein
the main body part is configured such that a mount surface for the operating means is set at a tilt angle tilted toward the grasping portion by a predetermined angle.

6. The operation apparatus according to claim 5, wherein
the tilt angle is set such that an angle defined by a flat surface of the mount surface for the operating means and the center line of the grasping portion is an angle smaller than 90°.

7. The operation apparatus according to claim 5, wherein
the grasping portion includes a flat-shaped first receiving portion receiving a root portion of the thumb of the operator and a second receiving portion disposed on a side surface opposite to the first receiving portion and receiving a finger of the operator other than the thumb, and
the main body part has a first mount surface arranged on a side surface positioned on the side of the first receiving portion and a second mount surface arranged on a side surface positioned on the side of the second receiving portion.

8. The operation apparatus according to claim 5, wherein
one of a trackball and an operation button indicating a bending direction is disposed on the first mount surface.

9. An operation apparatus for an endoscope having a bending operation apparatus for issuing an action instruction to a drive apparatus connected to a bending portion of the endoscope, comprising:
a main body part including an operation member whose proximal end is pivoted via a rotational movement supporting point in a rotational moving manner and which is supported so as to be tiltable between a central neutral position in a rotational movement range where the operation member rotationally moves about the rotational movement supporting point and a tilt position tilted from the neutral position;
a gasping portion which can be grasped by an operator;
an action member which acts in response to tilting of the operation member; and
an output portion which outputs an instruction signal changing according to action of the action member to a bending control apparatus controlling the drive apparatus, wherein
the operation member is configured such that the neutral position is set at a tilt angle tilted toward the grasping portion by a predetermined angle.

10. The operation apparatus for an endoscope according to claim 9, wherein
the tilt angle is set such that an angle defined by the center line of the operation member and the center line of the grasping portion is an angle smaller than 90° at the neutral position.

11. The operation apparatus for an endoscope according to claim 9, wherein
the grasping portion includes a flat-shaped first receiving portion receiving a root portion of the thumb of the operator and a second receiving portion disposed on a side surface opposite to the first receiving portion and receiving a finger of the operator other than the thumb, and
the first receiving portion is disposed on the side of a mount surface for the operation member of the main body part.

12. The operation apparatus for an endoscope according to claim 9, wherein
the main body part is configured such that a second mount surface on which operating means other than the operation member is mounted is disposed on a side surface positioned on the side opposite to a mount surface on which the operation member is mounted.

13. The operation apparatus for an endoscope according to claim 12, wherein
the second mount surface includes a suction switch controlling suction action of the endoscope, a gas-feed and water-feed switch controlling gas-feed and water-feed actions of the endoscope, and a video switch of the endoscope.

14. The operation apparatus for an endoscope according to claim 9, wherein
the operation member comprises a joystick instructing a bending direction of the bending portion,
the joystick has a finger receiving portion at a head portion of a stick shaft, and
the finger receiving portion is formed with a projecting portion arc-shaped along a direction corresponding to upward and downward bending directions of the bending portion and a recessed portion arc-shaped along directions corresponding to leftward and rightward bending directions of the bending portion.

15. The operation apparatus for an endoscope according to claim 9, wherein
the finger receiving portion has a rib for finger rest formed by further projecting a portion of the arc-shaped projecting portion in the arc-shaped recessed portion.

16. An operation apparatus for an endoscope having a bending operation apparatus for issuing an action instruction to a drive apparatus connected to a bending portion of the endoscope, comprising:
a main body part including operating means for instructing a bending direction of the bending portion;
a grasping portion which can be grasped by an operator;
an action member which acts in response to operation of the operating means; and
an output portion which outputs an instruction signal changing according to action of the action member to a bending control apparatus controlling the drive apparatus, wherein
the main body part is configured such that a mount surface for the operating means is set at a tilt angle tilted toward the grasping portion by a predetermined angle.

17. The operation apparatus for an endoscope according to claim 16, wherein
the tilt angle is set such that an angle defined by a flat surface of the mount surface for the operating means and the center line of the grasping portion is an angle smaller than 90°.

18. The operation apparatus for an endoscope according to claim 16, wherein
the grasping portion includes a flat-shaped first receiving portion receiving a root portion of the thumb of the operator and a second receiving portion disposed on a side surface opposite to the first receiving portion and receiving a finger of the operator other than the thumb, and
the main body part has a first mount surface arranged on a side surface positioned on the side of the first receiving portion and a second mount surface arranged on a side surface positioned on the side of the second receiving portion.

19. The operation apparatus for an endoscope according to claim 16, wherein
one of a trackball and an operation button indicating a bending direction is disposed on the first mount surface, and
the second mount surface includes a suction switch controlling suction action of the endoscope, a gas-feed and water-feed switch controlling gas-feed and water-feed actions of the endoscope, and a video switch of the endoscope are arranged on the second mount surface.
